# EUROPEAN PATENT APPLICATION

(11) **EP 1 048 312 A2**
(43) Date of publication of application: **02.11.2000**
(21) Application number: 00103724.1
(22) Date of filing: 22.02.2000
(51) Int. Cl.: A61M 16/00

(54) **Breathing gas supply apparatus**

(30) Priority: 27.04.1999 SE 9901510
(71) Applicant: Siemens-Elema AB, 171 95 Solna 1 (SE)
(72) Inventor: Cewers, Göran, 223 50 Lund (SE); Skog, Göran, 168 56 Bromma (SE)

(57) **Abstract**

A breathing gas supply apparatus comprises a variable volume reservoir (30) from which gas is dispensable and includes a section (42) moveable proportional to variations in volume of a gas containing portion of the reservoir (30). A position sensor (40) is provided and is adapted to monitor the location of the section (42) throughout an operating range of volumes of the variable volume reservoir (30). The position sensor (40) preferably comprises a non-ionising radiation, for example optical radiation, emitter (52) and a complementary detector (54) arrangement co-operable with the section (42) so that the section (42) will detectably influence the radiation from the emitter (52) incident on the detector (54) dependent on its location.

## Description

The present invention relates to a breathing gas supply apparatus and in particular to an apparatus comprising a variable volume reservoir for dispensing breathing gas, particularly anaesthetic gas for inspiration by a patient.

Aided breathing systems, for example patient respirators, ventilators and anaesthetic delivery systems, are generally known in which breathing gas is supplied from a variable volume container into a respiration circuit which carries gas to and from the airways of a patient.

One such system is described in US 5 678 540 and comprises a variable volume reservoir in the form of pressure-transmitting bellows, the internal volume of which is connected to the respiration circuit so as to be able to receive exhaled breathing gas from and re-supply it to a patient. The bellows is contained within a fixed volume container into which a pressurised drive gas may be admitted. With this so called "bag-and-bottle" arrangement an increased pressure over that within the bellows is provided within the fixed volume container during an inspiration phase of a patient breathing cycle. This increased pressure tends to collapse the bellows to transmit the pressure difference to the gas within and thereby deliver breathing gas into the respiration circuit. During an expiration phase of the patient breathing cycle the drive gas within the fixed wall container is removed through an outlet valve and a relative overpressure develops within the respiration circuit and bellows which causes the bellows to expand and receive exhaled breathing gas.

A position sensor is used to detect when the bellows approaches maximum expansion and then to provide a signal to control the operation of the outlet valve in order to minimise the pressure difference between the drive gas and the gas within the bellows device. The supply of breathing gas from the bellows device is controlled through the control of the supply of drive gas to the container and will stop when the a pressure equilibrium develops between the drive gas within the fixed volume container and the gas within the bellows device. Thus the inspiration phase is determined by the pressure and supply duration of the drive gas.

One problem with this known breathing system is that in order for it to properly function the bellows must always start the delivery of gas from its maximum expanded position as detected by the fixed position sensor. This may not always be the most useful position from which to start the delivery, for example the tubing and gas within the respiration circuit has a certain compressibility which must be overcome by the gas from the bellows so that there may be a delay between the supply of drive gas to compress the bellows and delivery of gas to the patient. This delay may be significant, especially in infant care where the compressible volume of the breathing system may approach or even exceed the volume of gas to be delivered to the patient.

Moreover, when used in a re-breathing circuit such as described in US 5 678 540 an amount of gas may be lost from the circuit, for example because of carbon dioxide (CO₂) elimination by the CO₂ absorber within the circuit, and an unknown amount of gas will enter the bellows. An amount of "fresh gas" may then be required to be introduced into the bellows in order to start its delivery of gas from the position detected by the position sensor. An abrupt change of the volume of the bellows occurs to return it to its working position which may result in a discontinuity in the supply of gas to the patient.

It is therefore an aim of the present invention to provide a breathing gas supply apparatus which can provide a greater flexibility in the selection of the operating range of volumes for the reservoir, over which gas may be supplied to the patient.

This is achieved by a breathing gas supply apparatus according to and characterised by the present Claim 1. By providing a position sensor that monitors of the location of the section throughout an operating range of volumes of the reservoir the reservoir volume may be monitored, preferably continuously. This enables the delivery of breathing gas to be started at any suitable volume of the reservoir within the operating range.

Moreover, since the volume of the gas delivered from the apparatus can be readily determined and controlled a greater flexibility in the operational uses of the reservoir may be provided for, for example the same reservoir may be used to supply adult or neo-natal patients.

Additionally, as only one position sensor is employed to monitor a plurality of locations of the section a cost benefit is obtained over known sensor arrangements where one sensor is provided for each location to be to monitored.

Usefully the position sensor may comprise a complementary non-ionising radiation emitter and detector disposed remote of the section which may be formed as at least part of a moveable end wall of a collapsible bellows device. Being remote of the variable volume container the effect of the physical presence of the sensor on the operation of the container is minimised.

Embodiments of the invention will now be described by way of example only and with reference to the drawings of the accompanying figures, of which:
Figure 1 shows a schematic representation of an aided breathing system including an apparatus according to the present invention.
Figure 2 shows an embodiment of a breathing gas supply apparatus according to the present invention and useable in the system of Figure 1.
Figure 3 shows a second embodiment of a breathing gas supply apparatus according to the present invention.
Figure 4 shows a further embodiment of a breathing gas supply apparatus according to the present invention.

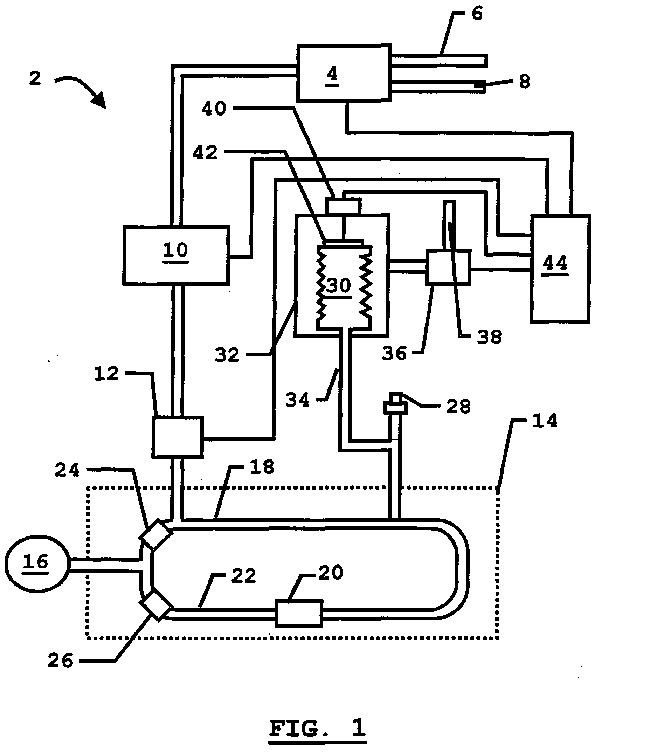

Considering Figure 1, an anaesthetic breathing system 2 is shown. The anaesthetic system 2 comprises a gas mixer 4, which receives nitrous oxide (N₂O) via a nitrous oxide connection 6 and oxygen (O₂) via an oxygen connection 8. N₂O and O₂ are mixed in defined proportions in the gas mixer 4, and the mixed gas is sent to a vaporiser device 10. An anaesthetic can be vaporised in the vaporiser device 10 and added to the gas mixture from the gas mixer 4 before the mixture is sent to a fresh gas reservoir 12. Breathing gas from the fresh gas reservoir 12 is sent to a breathing gas circuit 14. The breathing gas circuit 14 is connected to a patient 16 who, in this instance, receives a gas mixture containing an anaesthetic.

The breathing gas circuit 14 is a recirculating breathing gas circuit, i.e. the same breathing gas is reused immediately after expiration and returned to the patient through an inspiratory line 18. Expired gas passes from the patient 16 and through an expiratory line 22 in which there is situated a CO₂ absorber 20 which removes carbon dioxide from the expired gas. The direction of breathing gas flow in the breathing gas circuit 14 is governed by a first check valve 24 and a second check valve 26. Surplus gas in the breathing gas circuit 14 is evacuated via a pressure relief valve 28 which also ensures that pressure in the breathing gas circuit 14 and, accordingly, in the lungs of the patient 16, does not become excessive. The removed gas is preferably sent to some form of evacuation device (not shown in the figure).

The breathing cycle, which comprises an inspiration phase and an expiration phase, of the patient 16 is controlled by controlling the operation of a variable volume reservoir, here shown in the form of a bellows 30 placed in a container 32. The gas space in the bellows 30 is connected to the breathing gas circuit 14 via a gas line 34, and the space between the bellows 30 and the wall of the container 32 is connected to a drive gas unit 36 which receives a drive gas through a drive gas connection 38. When an inspiration is to be imposed on the patient 16, the space between the bellows 30 and the wall of the container 32 is filled, compressing the bellows 30 and forcing breathing gas through the inspiratory line 18 and to the patient 16. During expiration, gas is released from the space between the bellows 30 and the wall of the container 32 under control of the drive gas unit 36, whereupon gas flows into the bellows 30 via the expiratory line 22 and the gas line 34.

This "bag 30 and bottle 32" breathing gas supply apparatus also includes a position sensor 40 for continuously monitoring the position of the bellows top 42 and for outputting a signal to a control device 44 indicative of the monitored position.

The control device 44, which conveniently comprises a suitably conditioned microprocessor, controls and regulates the supply of breathing gas within the anaesthetic system 2. The control device 44 therefore regulates the mixture of nitrous oxide and oxygen in the gas mixer 4, and the supply of anaesthetic to the vaporiser device 10. Signal pathways between the control device 44 and other components 4,10,12,36,40 of the breathing system 2 are also shown in Figure 1.

Additionally the control device 44 regulates the patient's breathing cycles via the drive gas unit 36. To do this, the control device 44 is conditioned to use the output from the sensor 40 to determine when a desired volume of breathing gas has been delivered to the patient 16 and control the drive gas unit 36 so as to provide a desired flow of gas during an inspiration phase of the breathing cycle. In this manner the entire volume of gas within the bellows 30 need not be delivered to the patient so that the same variable volume container 30 may be used to provide breathing gas to patients 16 having different lung volumes. Moreover, the control device 44 may be conditioned to operate the bellows 30 at volumes away from its maximum volume in order to accommodate compliance within the gas tubes of the breathing system 2. This provides a breathing system 2 with a more rapid response to changes in delivery volumes or between expiration and inspiration phases as compared with known systems. This is because in known systems a part of the volume of the gas delivered from the bellows 30 is used to accommodate the compressible volume of both the gas and the gas tubing before gas is supplied to the patient 16.

Furthermore the control device 44 can regulate the supply of fresh breathing gas to the breathing gas circuit 14 from the fresh gas reservoir 12 so as to compensate for any gas lost, for example removed in the CO₂ absorber 20 or used by the patient 16, during each breathing cycle. To do this the control device 44 is programmed to use the output from the position sensor 40 to determine the volume of breathing gas lost during each breathing cycle. This may be achieved by determining the difference between the expanded position of the bellows 30, as determined using the output from the position sensor 40, at the beginning and end respectively of consecutive inspiration and expiration phases. The supply of fresh breathing gas from the reservoir 12 can then be regulated to compensate for the so determined difference.

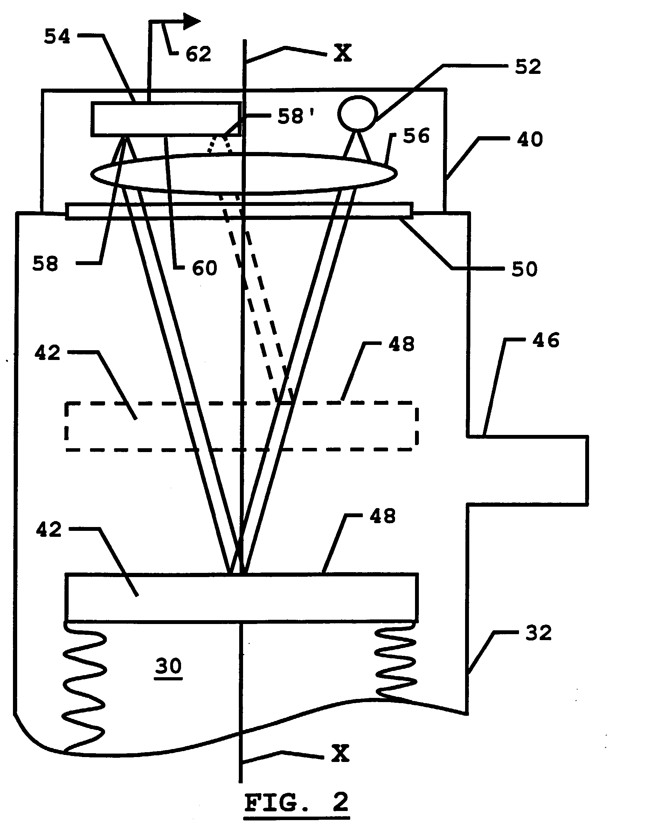

The operation of the position sensor 40 may be better understood with reference to the embodiment of a breathing gas supply apparatus shown in Figure 2 in which components common with Figure 1 have the same reference numerals as in that figure. Figure 2 shows an upper part of a breathing gas supply apparatus which comprises a variable volume reservoir in the form of a collapsible bellows 30, a rigid walled container 32 for housing the bellows and a position sensor 40. The bellows 30 is arranged to collapse and expand along an axis X in response variations in the pressure of a driving gas within the container 32 which enters and exits through an access port 46. A bellows top 42 is provided with a light reflective surface 48 which moves as the bellows top 42 moves. This surface 48 is shown in Figure 2 in two different positions along the X axis as represented by solid and broken line constructions of the bellows top 42.

The position sensor 40 is mounted to the outside of the container 32 adjacent a transparent top section 50 of the container 32 and comprises a light emitter 52 and detector 54 arrangement. The light emitter 52 is disposed so as to be able to continuously illuminate the reflective surface 48 of the bellows top 42 as its position changes along the axis X. The detector 54 is arranged to detect light from the emitter 52 after reflection from the reflective surface 48. A focussing lens 56 is positioned between the transparent section 50 and the emitter 52 and detector 54 which are arranged laterally displaced from one another in the focal plane of the lens 56. Thus, the emitter 52 is co-operatively disposed with the lens 56 to illuminate the surface 48 of the bellows top 42 with a substantially parallel beam of light at an oblique angle and the detector 54 is co-operatively disposed with the lens 56 to receive a focussed beam of light after reflection from the surface 48 of the bellows top 42. The single lens 56 may, of course, be replaced by individual lens systems for each of the emitter 52 and the detector 54.

The reflected beam is focussed by the lens 56 onto different regions 58,58' of a photosensitive surface 60 of the detector 54 dependent on the position of the reflective surface 48 of the bellows top 42 along the X axis (as illustrated by the broken line construction of the top 42). By providing a detector 54 which generates an output 62 indicative of the region 58,58' of the surface 60 onto which light has been focussed the position of the bellows top 42 and/or the volume of the gas containing portion of the bellows 30 may be calculated for use by the control device 44 (shown in Figure 1). This may be achieved, for example, by forming the photosensitive surface 60 as a conventional linearly segmented charge coupled device or a conventional position sensitive photodetector in operable connection to suitable electronic circuitry similar to detectors used in the focus control of autofocussing cameras and then providing a look up table of detected incident position 58,58' against location of bellows top 48 within a memory of the control device 44 or by employing an algorithm representative of the same within the device 44. The microprocessor of that device 44 is then programmed to calculate the position and/or volume from the output signal 62 of the detector 54 using the table or algorithm.

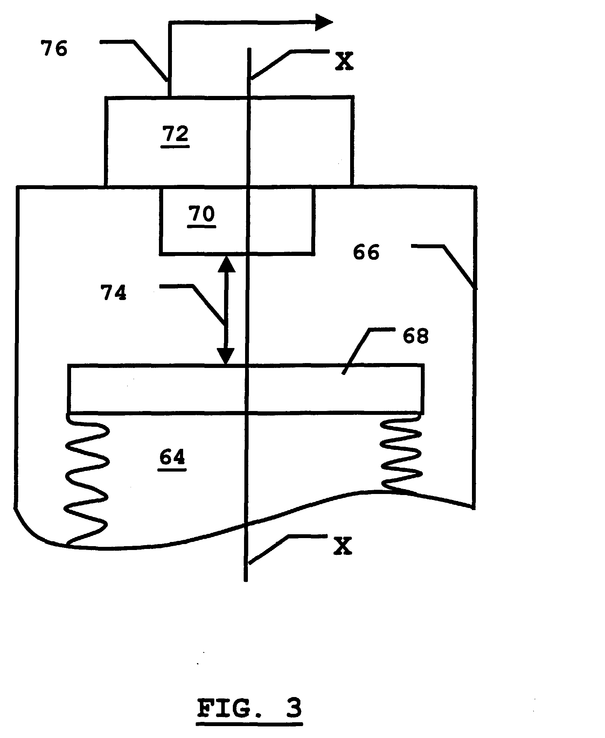

A second embodiment of a breathing gas supply apparatus is shown in Figure 3 which comprises a bellows 64 collapsible and expandable along an axis X within a container 66 under the influence of a drive gas in a manner similar to that described above. The bellows 64 is provided with a top 68 that moves along the axis X proportional to changes in the gas containing volume of the bellows 64. An ultrasound transducer 70 is also provided to act as a position sensor to measure the location of the bellows top 68 along the axis X using conventional sonar ranging techniques.

As shown in Figure 3, the transducer 70 is mounted within the container 66 and is operable to emit ultrasound towards the top 68 and to detect that part of the emitted ultrasound reflected from the top 68. An analyser/control unit 72 is connected to the transducer 70 to cycle its operation as an emitter and a detector and to analyse the detected ultrasound to determine distance between the transducer 70 and bellows top 68 using transit time or phase measurement techniques, common to art of sonar ranging. The so determined distance and/or a volume of the gas holding portion of the bellows 64 derived from the distance is then provided as an output 76 useable within the control device 44 (not shown) as described with respect to Figure 1.

It will be appreciated by those skilled in the art that the sonar ranging technique may also be employed in embodiments where the transceiver 70 is replaced with separate emitter and detector elements and further in embodiments where one or other of the elements is disposed so as to move with the bellows top 68.

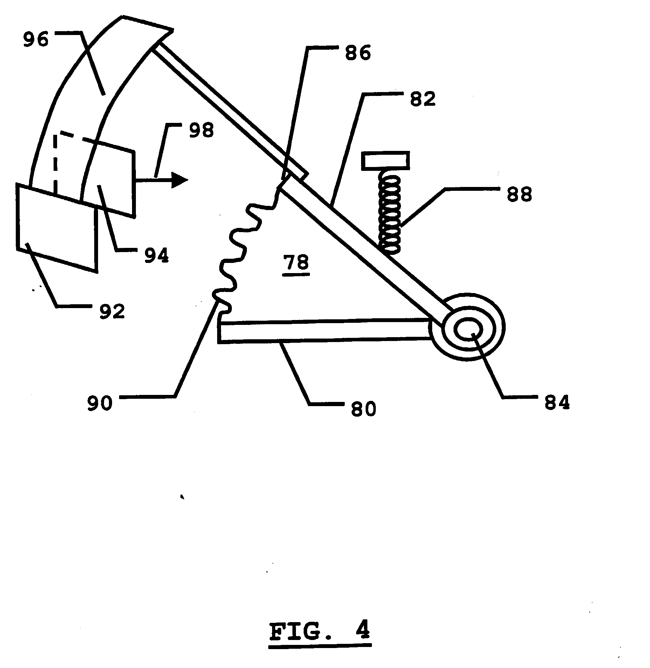

A further breathing gas supply apparatus according to the present invention is shown in Figure 4. A variable volume container 78 has a bottom fixed wall 80 and a moveable top wall 82. The bottom wall 80 and top wall 82 are pivotally connected at one end 84 so that the free end 86 of top wall 82 will describe an arc as it moves under the influence of a spring driving means 88 to collapse and expand a flexible wall 90.

A position sensor is provided which comprises a spaced apart complimentary optical radiation emitter 92 and detector 94 and a moveable graduated density neutral density filter 96, disposed between the emitter 92 and detector 94 to provide a variable attenuation for the radiation passing between them. The neutral density filter 96 is connected to the free end 86 of the bellows top 82 to move between the emitter 92 and detector 94 as the end 86 moves and is provided with a predetermined gradation of neutral density so that the intensity of optical radiation detected by the detector 94 varies with a known relationship to the variation in the position of the end 86. The detector 94 is then arranged to generate an output 98 proportional to the detected intensity of radiation from which the location of the end 86 and/or the volume of the gas containing portion of the variable volume container 78. The position sensor 92,94,96 can be easily calibrated by measuring the intensity of radiation passing through the filter 96 at the extreme working positions of the variable volume container 78 (e.g. fully expanded and fully collapsed positions) and from a knowledge of the gradation of the filter 96.

## Claims

1. A breathing gas supply apparatus comprising a variable volume reservoir (30;64;78) from which gas is dispensable, the reservoir (30;64;78) includes a section (42;68;82) moveable proportional to variations in volume of a gas containing portion of the reservoir; and a position sensor (40;70,72;92,94,96) having an output (62;76;98) dependent on the location of the section (42;68;82) **characterised in that** the position sensor (40;70,72;92,94,96) is adapted to monitor the location of the section (42;68;82) at a plurality of locations throughout an operating range of volumes of the variable volume reservoir (30;64;78).

2. A system as claimed in Claim 1 **characterised in that** the position sensor (40;70,72;92,94,96) includes a non-ionising radiation emitter (52;70;92) and a detector (54;70;94) adapted to provide an output responsive to radiation from the emitter (52;70;92) incident thereon; and in that the section (42;68;82) is co-operable with the position sensor (40;70,72;92,94,96) to detectably influence the incident emitted radiation in dependence of the location of the section.

3. A system as claimed in Claim 2 **characterised in that** the emitter (52) and detector (54) operate with optical radiation and are disposed remote from the section (42) with the detector (54) being arranged to detect emitted radiation reflected from a face (48) of the section (42).

4. A system as claimed in Claim 3 **characterised in that** the emitter (52) and detector (54) co-operate with the section (42) to define a flight path for the radiation which is displaceable across a photosensitive surface (60) of the detector (54) with changes in the location of the section (42) and in that detector (54) is adapted to generate an output dependent on the incident position (58,58') of the radiation on the photosensitive surface (60).

5. A system as claimed in Claim 4 **characterised in that** the there is further provided a lens system comprising one or more lenses (56) adapted to provide a substantially parallel beam of radiation from the emitter (52) at an oblique angle to the face (48) of the section (42) and to converge the reflected beam on to a different portion of the photosensitive surface (60) of the detector (54) dependent on the location of the section (42).

6. A system as claimed in Claim 5 **characterised in that** the emitter (52) comprises an effective point source located co-planar with the photosensitive surface (60) in a focal plane of a common lens (56).

7. A system as claimed in any of the Claims 4 to 6 **characterised in that** the detector (54) comprises a linearly segmented charge coupled device.

8. A system as claimed in Claim 2 **characterised in that** the emitter (70) and detector (70) operate with ultrasonic radiation.

9. A system as claimed in Claim 8 **characterised in that** the emitter and detector comprise a transceiver (70) disposed remote from the section (68) and arranged to detect emitted radiation reflected from a face of the section (68).

10. A system as claimed in claim 2 **characterised in that** the emitter (92) and detector (94) operate with optical radiation and are located to transmit radiation therebetween along a fixed flight path at a site remote from the section (82) and in that the position sensor further comprises a graduated density neutral density filter (96) movable in the flight path between the emitter (92) and detector (94) in response to the movement of the section (82) to variably attenuate the emitted radiation incident on the detector (94).
